# EUROPEAN PATENT SPECIFICATION

(11) **EP 1 922 054 B1**
(45) Date of publication and mention of the grant of the patent: **28.03.2012**
(21) Application number: 05770871.1
(22) Date of filing: 17.06.2005
(51) Int. Cl.: A61K 8/64, A61Q 5/12, A61Q 19/08, A61Q 19/10, A61Q 17/04

(54) **Cosmetic compositions containing protein hydrolysates**
Proteinhydrolysate enthaltende kosmetische Zusammensetzungen
Compositions cosmétiques contenant d'hydrolysats de protéine

(30) Priority: 28.06.2004 EP 04015155
(43) Date of publication of application: 21.05.2008
(73) Proprietor: DSM IP Assets B.V., 6411 TE Heerlen (NL)
(72) Inventor: VOLLHARDT, Juergen H., CH 4433 Ramlinsburg (CH); MAILLAN, Philippe Emmanuel, F-68440 Eschentzwiller (FR)
(74) Representative: Best, Michael
(86) International application number: PCT/EP2005/006556
(87) International publication number: WO 2006/000350

(56) References cited:
- EP-A- 0 979 829
- WO-A-02/45523
- WO-A-03/102195
- US-A1- 2004 120 918
- US-B1- 6 358 929

## Description

The present invention is directed to cosmetic compositions containing specific protein hydrolysates which are rich in carboxy terminated proline. The cosmetic compositions are particularly useful for treating wrinkles and moisturizing the skin.

Human skin undergoes certain normal cornification processes which give the skin its characteristic appearance. Casual factors or external factors such as a raw climate, wind, photo-damage and irritation triggered by the sun, rain and snow, however, disturb this normal condition of the skin, and there appears a roughness, a formation of scales (for example on the scalp), an excessive keratinization and similar phenomena. Furthermore, in the course of aging of the skin various signs appear that are especially reflected by a change in the structure and function of the skin. One of these signs is the appearance of fine lines and deep wrinkles, the size and number of which increases with age. The microrelief of the skin becomes less uniform and is of unisotropic nature. In parallel with age the skin becomes more sensitive towards disturbing influences, either intrinsic or extrinsic, which may result in itch, redness or even darker spots, particular on hands and the facial area due to pigmentation disorders. These unwanted signs may lead to an undesired age judgment of a person.

Cosmetic preparations are essentially useful for skin care. One aim of skin care in the cosmetic sense is to strengthen or rebuild the skin's natural function as a barrier against environmental influences (e.g. UV-light, dirt, chemicals, microorganisms) and against the loss of endogenous substances (e.g. water, natural lipids, electrolytes). If this function becomes impaired, increased resorption of toxic or allergenic substances or attack by microorganisms may result, leading to toxic or allergic skin reactions.

Another aim of skin care is to compensate for the loss by the skin of lipids and water caused by daily washing. This is particularly important, if the natural regeneration ability is inadequate. Furthermore, skin care products should protect against environmental influences, in particular against sun and wind, and delay skin aging. Also dry air conditions which occur particularly in winter tend to reduce the moisture content of the skin. Already this effect leads to a reduced smoothness and a dull skin surface. It is therefore important for a cosmetic skin care product to replenish the skin moisture content and to provide ingredients preventing the loss of moisture. Furthermore dry skin is prone more deleterious effects leading to age phenomena and sensory discomfort, like itching or stinging. It is therefore one goal of the present invention to provide safe materials to counteract these effects.

Strengthening or thickening of the epidermis together with an optimized skin barrier lipid synthesis can rebuild the skin's barrier ability and is therefore of significant cosmetic value. Reduced transepidermal water loss (TEWL) is a sign of an intact lipid barrier, which acts also as first defense line to protect against the appearance of skin wrinkles.

Another strategy to fight wrinkles is to stimulate the collagen synthesis in the dermis. A number of degenerative processes act on the collagen matrix and is triggered by extrinsic factors like UV radiation, pollution in general and particular cigarette smoke or intrinsic factors leading to any chronic or subchronic inflammation. Destruction and/or impaired repair efficacy leads to a more dense and less elastic macro structure of the dermis, which in turn leads to the formation of deep wrinkles. Enhancing the de novo synthesis of collagen or other structural proteins of the dermis is considered a valuable therapy to reduce the existing wrinkles and to protect against the appearance of new wrinkles.

Of particular importance for anti-aging cosmetics is to inhibit the senescence of skin cells in order to keep their regular metabolic level on a constant and beneficial level.

There exists a wide variety of literature regarding cosmetic preparations, in particular regarding cosmetic preparations for treating wrinkles. As examples of the extensive literature it can be referred e.g. to GB 906,000, EP-A 699 429 or WO 03/086342.

It is known to include enzymatic hydrolysates of proteins (protein hydrolysates) into cosmetic compositions for achieving cosmetic effects. US-B 6,506,732 discloses hydrolysates of milk proteins and their use in cosmetic and pharmaceutical formulations. The hydrolysates are said to have skin hydrating and skin regenerating properties. American Journal of Clinical Dermatology (2002), 3 (8), 547-55 discloses the use of hydrolyzed proteins in nail hardeners. Protein hydrolysates are also added to hair care products to repair broken hair, to soaps, bath gels, creams, etc. (Allergy (Nov. 1998), 53 (11), 1078-82).

However, the use of protein hydrolysates in cosmetic formulations has been criticized, because protein hydrolysates can cause allergic reactions. Contact Dermatitis (June 2000) 42 (6), 360 discloses that hydrolyzed wheat protein in cosmetic creams can be responsible for an allergic contact dermatitis. Allergy (November 1998) 53 (11), 1078-82 tested 22 protein hydrolysates in hair care products, and it was found that protein hydrolysates in hair cosmetic can cause contact urticaria, especially in patients with atopic dermatitis. Archive of Dermatology (July 1976), 112 (7), 1008-9 reports of a 21 year old woman who developed a severe dermatitis of the face after using a proprietary skin cleanser containing TEA-Coco hydrolyzed protein. A reaction was also found with other condensates of fatty acids and protein hydrolysates.

It has been suggested to reduce the allergenicity of protein hydrolysates by chemically modifying the hydrolysates e.g. by substituting one or more amino acid residues with other amino acid residues and/or coupling the proteins with polymers. However, such modifications can change the effectiveness of the protein and the products can no longer be marketed as "natural" products.

US-B 6,506,732 discloses a specific hydrolysate of protein which is said to have a reduced antigenicity.

EP 979829 A2 discloses cosmetic or pharmaceutical compositions comprising oligopeptides for treatment of unwanted skin-pigmentation.

US 2004/0120918 A1 discloses cosmetics including a polypeptide of between 3 and 12 amino acids in length and a ceramide.

US-B 6,358,929 discloses cosmetic compositions containing a peptide containing the sequence Lysine-Proline-Valine.

The problem to be solved by the present invention is providing cosmetic compositions having excellent cosmetic properties which can usually be achieved by adding protein hyrolysates to cosmetic compositions but which have a low or no risk of causing allergic reactions by consumers. The compositions should have a favorable balance between cosmetic effects and risk of skin adverse reactions. Moreover the invention provides low odor and low color hydrolysis products, which are compatible with most cosmetic formulations including low fragrance containing products.

This problem is solved on the basis of the unexpected finding that certain protein hydrolysates which have a high content of peptides with a terminal proline and which up to now were only used in food applications or similar applications can advantageously be incorporated into cosmetic compositions. Cosmetic compositions which contain these protein hydrolysates have the advantageous properties of cosmetic compositions containing protein hydrolysates but they do not cause skin irritation with patients.

Therefore, in its broadest aspect the present invention provides a cosmetic composition containing a protein hydrolysate which has a high content of peptides with a terminal protein and a cosmetically acceptable excipient.

The protein hydrolysate is a protein hydrolysate which comprises peptides, wherein the molar fraction of peptides (%) carrying a carboxy terminal proline is more than two times higher than the molar fraction (%) of proline in the protein substrate used to generate the hydrolysate.

The protein hydrolysate is furthermore preferably
- a whey hydrolysate which comprises peptides wherein the molar fraction of peptides carrying a carboxy terminal proline is at least 8%, preferably at least 15%, more preferably from 30 to 70% and/or
- a casein hydrolysate which comprises peptides wherein the molar fraction of peptide carrying a carboxy terminal proline is at least 25%, preferably at least 30% and more preferably less than 70% and/or
- a soy hydrolysate which comprises peptides wherein the molar fraction of peptide carrying a carboxy terminal proline is at least 20%, preferably from 30 to 70% and/or
- a gluten hydrolysate which comprises peptides wherein the molar fraction of peptide carrying a carboxy terminal proline is at least 20%, preferably at least 30%, advantageously less than 70% and/or
- a barley hydrolysate which comprises peptides wherein the molar fraction of peptide carrying a carboxy terminal proline is at least 20%, preferably at least 30%, advantageously less than 70%.

Furthermore is preferred that the protein hydrolysate having a high content of peptides with a terminal proline is a protein hydrolysate which is rich in tripeptides, whereby the tripeptides are rich in proline at one end of the peptide.

The protein hydrolysates for use in the cosmetic compositions of the present invention are well-known in the prior art. The
- whey hydrolysate which comprises peptides wherein the molar fraction of peptides carrying a carboxy terminal proline is at least 8%, preferably at least 15%, more preferably from 30 to 70%;
- casein hydrolysate which comprises peptides wherein the molar fraction of peptide carrying a carboxy terminal proline is at least 25%, preferably at least 30% and more preferably less than 70%;
- soy hydrolysate which comprises peptides wherein the molar fraction of peptide carrying a carboxy terminal proline is at least 20%, preferably from 30 to 70%;
- gluten hydrolysate which comprises peptides wherein the molar fraction of peptide carrying a carboxy terminal proline is at least 20%, preferably at least 30%, advantageously less than 70% and
- barley hydrolysate which comprises peptides wherein the molar fraction of peptide carrying a carboxy terminal proline is at least 20%, preferably at least 30%, advantageously less than 70%,
and a process for producing such protein hydrolysates are known from WO 02/45523 and WO 02/45524. The protein hydrolysate is preferably a protein hydrolysate which comprises peptides, wherein the molar fraction of peptides (%) carrying a carboxy terminal proline is more than two times higher than the molar fraction (%) of proline in the protein substrate used to generate the hydrolysate, and such a protein hydrolysate is also known from these documents. Reference is made to these documents in particular regarding the preparation of the protein hydrolysates which is described in the documents in detail.

Protein hydrolysates which are rich in tripeptides, whereby the tripeptides are rich in proline at one end of the peptide, are known from WO 03/102195 . It is referred to WO 03/102195 in particular regarding the process for producing the protein hydrolysates which are rich in tripeptides, whereby the tripeptides are rich in proline at one end of the peptide, and regarding further preferred protein hydrolysates. The preferred protein hydrolysates of the present invention are also the preferred protein hydrolysates disclosed in WO 03/102195.

Protein hydrolysates which can be used for the cosmetic compositions of the present invention can also be prepared with the novel proteolytic enzymes disclosed in WO 02/068623.

Thus, the present invention provides cosmetic compositions containing a protein hydrolysate with a high content of terminal proline which is known from WO 03/102195, WO 02/45523 and/or WO 02/45524.

Particularly preferred are protein hydrolysates which are rich in tripeptides, whereby the tripeptides are rich in proline at one end of the peptide, wherein the peptide has a carboxy terminal proline as disclosed in WO 03/102195. These hydrolysates may optionally also comprise dipeptides. More preferred is a protein hydrolysate which is rich in tripeptides, wherein the tripeptides are rich in proline at one end of the peptide, preferably the carboxyl end of the peptide, wherein at least 20 molar%, more preferably at least 25 molar%, still more preferably at least 30 molar% of the peptides having a molecular weight of 200 to 2000 Dalton is present in the hydrolysate as tripeptide. Preferred is also a protein hydrolysate as defined above, wherein preferably at least 20%, more preferably at least 30%, still more preferably at least 40% of the proline present in the starting protein is present in the tripeptides. Preferred are also the protein hydrolysates as defined above, wherein at least 30% of the tripeptides or preferably at least 35% of the tripeptides have a carboxy terminal proline. Preferred are also the protein hydrolysates as defined above, wherein at least 70 molar% of the peptides, more preferably at least 75 molar% of the peptides, contain 2 to 7 amino acid residues (dipeptide to heptapeptide). Other preferred protein hydrolysates are known from WO 03/102195.

Preferred are also precursor products made on the basis of above mentioned hydrolysates, particular those, which are acylated at the N-terminus of the amino acid. Such precursor products have an increased hydrophobicity or skin penetration. Furthermore, by acylation another cosmetically active part can be added to the peptide moiety. Particularly preferred is alkylacylation with linear or branched, saturated or unsaturated fatty acids, preferably with fatty acids having 1 to 30 carbon atoms. More preferred are palmitoyl or phytanoyl derivatives. The term "protein hydrolysates" as used in this specification also encompasses the above precursor products.

Referring now to the
- whey hydrolysate which comprises peptides wherein the molar fraction of peptides carrying a carboxy terminal proline is at least 8%, preferably at least 15%, more preferably from 30 to 70%
- casein hydrolysate which comprises peptides wherein the molar fraction of peptide carrying a carboxy terminal proline is at least 25%, preferably at least 30% and more preferably less than 70%
- soy hydrolysate which comprises peptides wherein the molar fraction of peptide carrying a carboxy terminal proline is at least 20%, preferably from 30 to 70%
- gluten hydrolysate which comprises peptides wherein the molar fraction of peptide carrying a carboxy terminal proline is at least 20%, preferably at least 30%, advantageously less than 70% and
- barley hydrolysate which comprises peptides wherein the molar fraction of peptide carrying a carboxy terminal proline is at least 20%, preferably at least 30%, advantageously less than 70%,
and the protein hydrolysate which is a protein hydrolysate which comprises peptides, wherein the molar fraction of peptides (%) carrying a carboxy terminal proline is more than two times higher than the molar fraction (%) of proline in the protein substrate used to generate the hydrolysate and which are disclosed in WO 02/45523 and in WO 02/45524, the following preferred embodiments should be noted.

Preferably, the protein hydrolysate as defined above comprises peptides, wherein the molar fraction of peptides (%) carrying a carboxy terminal proline is at least two times, more preferably at least three times, the molar fraction (%) of proline in the protein substrate used to produce the hydrolysates. Preferably, the average length of the peptides in the protein hydrolysates is from 3 to 9 amino acids. Preferred hydrolysates are a whey hydrolysate which comprises peptides wherein the molar fraction of peptides carrying a carboxy terminal proline is at least 8%, preferably at least 15%, more preferably from 30 to 70%, a casein hydrolysate which comprises peptides wherein the molar fraction of peptide carrying a carboxy terminal proline is at least 25%, preferably from 30 to 70%, and a soy hydrolysate which comprises peptides wherein the molar fraction of peptides carrying a carboxy terminal proline is at least 20%, preferably from 30 to 70%. Preferred are protein hydrolysates, wherein at least 10% of the protein substrate is hydrolyzed, preferably wherein from 20 to 90% of the protein substrate is hydrolyzed. By peptides or peptide fragments it is preferably meant peptides with molecular masses from 400 to 2000 Dalton. These peptides can be analyzed according to the LC/MC analysis as described in WO 02/45523.

In general in the production of the protein hydrolysates the protein substrate is substantially hydrolyzed, advantageously for at least 50%. Preferably at least 10% of the protein substrate is converted into peptides having molecular masses from 400 to 2000 Dalton.

More preferably from 20 to 90% and even more preferably from 30 to 80% of the protein substrate is converted into such peptides.

In one embodiment, a protein substrate may be incubated with an enzyme mixture comprising an isolated, purified proline-specific endoprotease, a serine endoprotease or a metallo endoprotease and a carboxypeptidase to produce a protein hydrolysate enriched in peptide fragments having a carboxy terminal proline as disclosed in WO 02/45523.

The enzyme mixture of WO 02/45523 which is preferably used in the present invention is characterized in that it contains at least one endoprotease for example a serine protease or a metallo endoprotease in conjunction with a proline-specific endoprotease (E. C. 3.4..21.26) to provide a primary hydrolysate. More specifically, an isolated, purified proline-specific endoprotease and a serine protease or metallo protease enzyme mixture capable of producing a protein hydrolysate comprising peptide fragments, wherein at least 8%, preferably at least 15%, more preferably from 30 to 70% of said peptide fragments have a carboxy terminal proline as disclosed in WO 02/45523 is used.

Preferred is also a protein hydrolysate enriched with a relatively high content of peptides having proline as the carboxy terminal amino acid residue. Such enriched hydrolysates may comprise at least 8%, preferably at least 15%, more preferably from 30 to 70% peptide fragments having a carboxy terminal proline residue. These protein hydrolysates can be obtained by enzyme preparations which are capable of generating peptides bearing proline residues at carboxy termini, which are disclosed in WO 02/45523.

Most preferred are protein hydrolysates which can be obtained by a process corresponding to the process of example 1 of WO 02/45523, using a suitable substrate. Preferred are also mixtures of the protein hydrolysates disclosed in WO 02/45523 and in WO 03/102195.

Substrates for hydrolysis by an enzyme mixture include whole milk, skimmed milk, acid casein, rennet casein, acid whey products or cheese whey products. The *Aspergillus* derived proline-specific endoprotease does not only cleave at the carboxy terminal side of proline residues but also at the carboxy terminal side of hydroxyproline residues which makes other, collagen-based animal proteins such as gelatin as well as bones or fishbones containing residual meat, interesting substrates for the enzyme. Moreover, vegetable substrates like wheat gluten, milled barley and protein fractions obtained from, for example, soy, rice or corn are suitable substrates. Milk protein hydrolysates produced according to WO 02/45523 may be used with or without additional filtration or purification steps.

Unexpectedly, the above-defined protein hydrolysates which have a high content of peptides with a terminal proline, have excellent activity in various cosmetic applications, while not causing the irritations of the skin which are caused by other protein hydrolysates produced by state of the art methods. The cosmetic compositions containing the protein hydrolysates as defined above show activity in particular for treating and preventing wrinkles, moisturizing the skin and hair as well as thickening of the epidermis but also for ameliorating the effect of aging of the skin which may be caused by external or environmental hazards or by the natural aging of the skin. Particular suitable are formulations with a high protein hydrolysate concentrations giving pronounced effects, e.g. above 0.1 % dry matter protein material, which can be formulated according to the present invention with no or low risk of adverse reactions.

The protein hydrolysates can also be used in cleaning or washing compositions or skin cleansers, particular those which are designed to be mild to the skin, especially baby care formulations to provide a cleaning effect, accompanied with skin rehydration without causing adverse reactions.

The present invention also envisages cosmetic compositions with protein hydrolysates which are usually employed in foodstuffs and which in the usual tests for allergens in foodstuffs do not show any significant positive reaction. Such tests are known in the art and commercially available, e.g. from the company Tepnel BioSystems Limited Flintshire CH5 2NT, England but also from other companies. Typical tests are tests for gluten, milk proteins (Casein, βLG, BSA), peanut protein, soy protein and sesame.

The cosmetic compositions are in particular compositions which can be used for treatment or prophylaxis of wrinkles, a thickening of the epidermis, the inhibition of senescence of skin cells, or non-therapeutical treatment of photodamage.

The protein hydrolysates can also be used in sunscreen compositions and in skin and hair cleaning compositions, such as soaps, bath gels, shower gels, liquid soaps, shampoos, bath cubes, foam bathes, skin cleansers, etc. The protein hydrolysates can also be used in hair conditioners, hair treatment sera and styling gels in rinse off as well as in leave on applications.

The compositions of the present invention are topical compositions.

The term "cosmetic preparation" or "cosmetic composition" as used in the present application refers to cosmetic compositions as defined under the heading "Kosmetika" in Römpp Lexikon Chemie, 10th edition 1997, Georg Thieme Verlag Stuttgart, New York. Skin and hair cleaning compositions are also particularly included in the cosmetic compositions of the invention.

The compositions of the present invention contain the protein hydrolysate as defined above with cosmetically acceptable excipients or diluents. If nothing else is stated, the excipients, additives, diluents, etc. mentioned in the following are suitable for cosmetic compositions.

If nothing else is stated, in this application parts and percentages are per weight and are based on the weight of the composition.

The compositions of the present invention are topical compositions, such as liquid or solid oil-in-water emulsions, water-in-oil emulsions, multiple emulsions, microemulsions, PET-emulsions, bickering emulsions, hydrogels, alcoholic gels, lipogels, one or multiphase solutions, foams, ointments, plasters, suspensions, powders, crèmes, cleanser, soaps and other usual compositions, which can also be applied by pens, as masks or as sprays.

The compositions of the invention can also contain usual cosmetic adjuvants and additives, such as preservatives/ antioxidants, fatty substances/ oils, water, organic solvents, silicones, thickeners, softeners, emulsifiers, sunscreens, cosmetic actives antifoaming agents, moisturizers, fragrances, surfactants, fillers, sequestering agents, anionic, cationic, nonionic or amphoteric polymers or mixtures thereof, propellants, acidifying or basifying agents, dyes, colorants, pigments or nanopigments, e.g. those suited for providing a photoprotective effect by physically blocking out ultraviolet radiation, or any other ingredients usually formulated into cosmetics.

The composition of the present invention can also contain one or more additional pharmaceutically or cosmetically active ingredients, in particular bisabolol, alkyldiols such as 1,2-pentandiol, hexanediol or 1,2-octanediol, vitamins, panthenol, phytol, phytantriol, ceramides and pseudeceramides, amino acids and bioactive peptides, AHA acids, polyunsaturated fatty acids, plant extracts, DNA or RNA and their fragmentation products or carbohydrates.

Additionally the composition of the present invention may contain UV-A and UV-B filters. Examples of UV-B or broad spectrum screening agents, i.e. substances having absorption maximums between about 290 and 340 nm, which are preferred for combination with the compounds of the present invention, are the following organic and inorganic compounds:
Acrylates such as 2-ethylhexyl 2-cyano-3,3-diphenylacrylate (octocrylene, PARSOL® 340), ethyl 2-cyano-3,3-diphenylacrylate and the like;
   --- Camphor derivatives such as 4-methyl benzylidene camphor (PARSOL® 5000), 3-benzylidene camphor, camphor benzalkonium methosulfate, polyacrylamidomethyl benzylidene camphor, sulfo benzylidene camphor, sulphomethyl benzylidene camphor, therephthalidene dicamphor sulfonic acid and the like;
   --- Cinnamate derivatives such as octyl methoxycinnamate (PARSOL® MCX), ethoxyethyl methoxycinnamate, diethanolamine methoxycinnamate (PARSOL® Hydro), isoamyl methoxycinnamate and the like as well as cinnamic acid derivatives bond to siloxanes;
   --- p-aminobenzoic acid derivatives, such as p-aminobenzoic acid, 2-ethylhexyl p-dimethylaminobenzoate, N-oxypropylenated ethyl p-aminobenzoate, glyceryl p-aminobenzoate,
   --- Benzophenones such as benzophenone-3, benzophenone-4, 2,2', 4, 4'-tetrahydroxy-benzophenone, 2,2'-dihydroxy-4,4'-dimethoxybenzophenone and the like;
   --- Esters of Benzalmalonic acid such as di-(2-ethylhexyl) 4-methoxybenzalmalonate;
   --- Esters of 2-(4-ethoxy-anilinomethylene)propandioic acid such as 2-(4-ethoxy anilinomethylene)propandioic acid diethyl ester as described in the European Patent Publication EP 0895 776;
   --- Organosiloxane compounds containing benzmalonate groups as described in the European Patent Publications EP 0358584 B1, EP 0538431 B1 and EP 0709080 A1;
   --- Drometrizole trisiloxane (Mexoryl XL);
   --- Pigments such as microparticulated TiO₂, and the like. The term "microparticulated" refers to a particle size from about 5 nm to about 200 nm, particularly from about 15 nm to about 100 nm. The TiO₂ particles may also be coated by metal oxides such as e.g. aluminum or zirconium oxides or by organic coatings such as e.g. polyols, methicone, aluminum stearate, alkyl silane. Such coatings are well known in the art.
   --- Imidazole derivatives such as e.g. 2-phenyl benzimidazole sulfonic acid and its salts (PARSOL®HS). Salts of 2-phenyl benzimidazole sulfonic acid are e.g. alkali salts such as sodium- or potassium salts, ammonium salts, morpholine salts, salts of primary, sec. and tert. amines like monoethanolamine salts, diethanolamine salts and the like.
   --- Salicylate derivatives such as isopropylbenzyl salicylate, benzyl salicylate, butyl salicylate, octyl salicylate (NEO HELIOPAN OS), isooctyl salicylate or homomenthyl salicylate (homosalate, HELIOPAN) and the like.
   --- Triazine derivatives such as octyl triazone (UVINUL T-150), dioctyl butamido triazone (UVASORB HEB), bis ethoxyphenol methoxyphenyl triazine (Tinosorb S) and the like.

Examples of broad spectrum or UV A screening agents i.e. substances having absorption maximums between about 320 and 400 nm, which are preferred for combination with the compounds of the present invention are the following organic and inorganic compounds:
--- Dibenzoylmethane derivatives such as 4-tert. butyl-4'-methoxydibenzoylmethane (PARSOL® 1789), dimethoxydibenzoylmethane, isopropyldibenzoylmethane and the like;
--- Benzotriazole derivatives such as 2,2'-methylene-bis-(6-(2H-benzotriazole-2-yl)-4-(1,1,3,3,-tetramethylbutyl)-phenol (TINOSORB M) and the like;
--- phenylene-1,4-bis-benzimidazolsulfonic acids or salts such as 2,2-(1,4-phenylene)bis-(1H-benzimidazol-4,6-disulfonic acid) (Neoheliopan AP);
--- amino substituted hydroxybenzophenones such as 2-(4-Diethylamino-2-hydroxy-benzoyl)-benzoic acid hexylester as described in the European Patent Publication EP 1046391;
--- Pigments such as microparticulated ZnO or TiO₂ and the like. The term "microparticulated" refers to a particle size from about 5 nm to about 200 nm, particularly from about 15 nm to about 100 nm. The particles may also be coated by other metal oxides such as e.g. aluminium or zirconium oxides or by organic coatings such as e.g. polyols, methicone, aluminum stearate, alkyl silane. Such coatings are well known in the art.

As dibenzoylmethane derivatives have limited photostability it may be desirable to photostabitize these UV-A screening agents. Thus, the term "conventional UV-A screening agent" also refers to dibenzoylmethane derivatives such as e.g. PARSOL® 1789 stabilized by, e.g.,
--- 3,3-Diphenylacrylate derivatives as described in the European Patent Publications EP-A 0 514 491 and EP-A 0 780 119;
--- Benzylidene camphor derivatives as described in the US Patent No. 5,605,680;
--- Organosiloxanes containing benzmalonate groups as described in the European Patent Publications EP-A 0358584, EP-A 0538431 and EP-A 0709080.

The compositions of the present invention preferably contain one or more antioxidants/preservatives. Based on the invention all known antioxidants usually formulated into cosmetics can be used. Especially preferred are antioxidants chosen from the group consisting of amino acids (e.g. glycine, histidine, tyrosine, tryptophan) and their derivatives, imidazole (e.g. urocanic acid) and derivatives, peptides such as D,L-carnosine, D-carnosine, L-carnosine and derivatives (e.g. anserine), carotenoids, carotenes (e.g. α-carotene, β-carotene, lycopene) and derivatives, chlorogenic acid and derivatives, lipoic acid and derivatives (e.g. dihydrolipoic acid), aurothioglucose, propylthiouracil and other thiols (e.g. thioredoxine, glutathione, cysteine, cystine, cystamine and its glycosyl-, N-acetyl-, methyl-, ethyl-, propyl-, amyl-, butyl- and lauryl-, palmitoyl-; oleyl-, y-linoleyl-, cholesteryl- and glycerylester) and the salts thereof, dilaurylthiodipropionate, distearylthiodipropionate, thiodipropionic acid and its derivatives (ester, ether, peptides, lipids, nucleotides, nucleosides and salts) as well as sulfoximine compounds (such as buthioninsulfoximine, homocysteinsulfoximine, buthioninsulfone, penta-, hexa-, heptathioninsulfoximine) in very low compatible doses (e.g. pmol to µmol/kg), additionally (metal)-chelators (such as α-hydroxyfatty acids, palmic-, phytinic acid, lactoferrin), β-hydroxyacids (such as citric acid, lactic acid, malic acid), huminic acid, gallic acid, gallic extracts, bilirubin, biliverdin, EDTA, EGTA and its derivatives, unsaturated fatty acids and their derivatives (such as γ-linoleic acid, linolic acid, oleic acid), folic acid and its derivatives, ubiquinone and ubiquinol and their derivatives, vitamin C and derivatives (such as ascorbylpalmitate and ascorbyltetra-isopalmitate, Mg-ascorbylphosphate, Na-ascorbylphosphate, ascorbylacetate), tocopherole and derivates (such as vitamin-E-acetate), mixtures of nat. vitamin E, vitamin A and derivatives (vitamin-A-palmitate and -acetate) as well as coniferylbenzoat, rutinic acid and derivatives, α-glycosylrutin, ferulic acid, furfurylidenglucitol, carnosin, butylhydroxytoluene, butylhydroxyanisole, trihydroxybutyrophenone, urea and its derivatives, mannose and derivatives, zinc and derivatives (e.g. ZnO, ZnSO₄), selenium and derivatives (e.g. selenomethionine), stilbenes and derivatives (such as stilbenoxide, trans-stilbenoxide) and suitable derivatives (salts, esters, ethers, sugars, nucleotides, nucleosides, peptides and lipids) of the named active ingredients. One or more preservatives/antioxidants may be present in an amount about 0.01 wt.% to about 10 wt.% of the total weight of the composition of the present invention. Preferably, one or more preservatives/antioxidants are present in an amount about 0.1 wt.% to about 1 wt.%.

Typically topical formulations also contain surface active ingredients like emulsifiers, solubilizers and the like. An emulsifier enables two or more not miscible components to be combined homogeneously. Moreover, the emulsifier acts to stabilize the composition. Emulsifiers that may be used in the present invention in order to form O/W, W/O, O/W/O or W/O/W emulsions/microemulsions include sorbitan oleate, sorbitan sesquioleate, sorbitan isostearate, sorbitan trioleate, polyglyceryl-3-diisostearate, polyglycerol esters of oleic/isostearic acid, polyglyceryl-6 hexaricinolate, polyglyceryl-4-oleate, polyglyceryl-4 oleate/PEG-8 propylene glycol cocoate, oleamide DEA, TEA myristate, TEA stearate, magnesium stearate, sodium stearate, potassium laurate, potassium ricinoleate, sodium cocoate, sodium tallowate, potassium castorate, sodium oleate, and mixtures thereof. Further suitable emulsifiers are phosphate esters and the salts thereof such as cetyl phosphate (Amphisol^{®} A), diethanolamine cetyl phosphate (Amphisol^{®}), potassium cetyl phosphate (Amphisol^{®} K), sodium glyceryl oleate phosphate, hydrogenated vegetable glycerides phosphate and mixtures thereof. Furthermore, one or more synthetic polymers may be used as an emulsifier. For example, PVP eicosene copolymer, acrylates/C₁₀₋₃₀ alkyl acrylate crosspolymer, acrylates/steareth-20 methacrylate copolymer, PEG-22/dodecyl glycol copolymer, PEG-45/dodecyl glycol copolymer, and mixtures thereof. The preferred emulsifiers are cetyl phosphate (Amphisol^{®} A), diethanolamine cetyl phosphate (Amphisol^{®}), potassium cetyl phosphate (Amphisol^{®} K), PVP Eicosene copolymer, acrylates/C₁₀₋₃₀-alkyl acrylate crosspolymer, PEG-20 sorbitan isostearate, sorbitan isostearate, and mixtures thereof. The one or more emulsifiers are present in a total amount about 0.01 wt.% to about 20 wt.% of the total weight of the composition of the present invention. Preferably, about 0.1 wt.% to about 10 wt.% of emulsifiers are used.

The lipid phase of the topical compositions can advantageously be chosen from:
- mineral oils and mineral waxes;
- oils such as triglycerides of caprinic acid or caprylic acid, preferable castor oil;
- oils or waxes and other natural or synthetic oils, in an preferred embodiment esters of fatty acids with alcohols e.g. isopropanol, propylene glycol, glycerin or esters of fatty alcohols with carboxylic acids or fatty acids;
- alkylbenzoates; and/or
- silicone oils such as dimethylpolysiloxane, diethylpolysiloxane, diphenylpolysiloxane, cyclomethicones
and mixtures thereof.

Exemplary fatty substances which can be incorporated in the oil phase of the emulsion, micro-emulsion, oleo gel, hydrodispersion or lipodispersion of the present invention are advantageously chosen from esters of saturated and/or unsaturated, linear or branched alkyl carboxylic acids with 3 to 30 carbon atoms, and saturated and/or unsaturated, linear and/or branched alcohols with 3 to 30 carbon atoms as well as esters of aromatic carboxylic acids and of saturated and/or unsaturated, linear or branched alcohols of 3-30 carbon atoms. Such esters can advantageously be selected from octylpalmitate, octylcocoate, octylisostearate, octyldodecylmyristate, cetearylisononanoate, isopropylmyristate, isopropylpalmitate, isopropylstearate, isopropyloleate, n-butylstearate, n-hexyllaureate, n-decyloleat, isooctylstearate, isononylstearate, isononylisononanoate, 2-ethyl hexylpalmitate, 2-ethylhexyllaurate, 2-hexyldecylstearate, 2-octyldodecylpalmitate, stearylheptanoate, oleyloleate, oleylerucate, erucyloleate, erucylerucate, tridecylstearate, tridecyltrimellitate, as well as synthetic, half-synthetic or natural mixtures of such esters e.g. jojoba oil.

Other fatty components suitable for use in the topical compositions of the present invention include polar oils such as lecithins and fatty acid triglycerides, namely triglycerol esters of saturated and/or unsaturated, straight or branched carboxylic acid with 8 to 24 carbon atoms, preferably of 12 to 18 carbon-atoms whereas the fatty acid triglycerides are preferably chosen from synthetic, half synthetic or natural oils (e.g. cocoglyceride, olive oil, sun flower oil, soybean oil, peanut oil, rape seed oil, sweet almond oil, palm oil, coconut oil, castor oil, hydrogenated castor oil, wheat oil, grape seed oil, macadamia nut oil and others); apolar oils such as linear and/ or branched hydrocarbons and waxes e.g. mineral oils, vaseline (petrolatum); paraffins, squalane and squalene, polyolefins, hydrogenated polyisobutenes and isohexadecanes, favored polyolefins are polydecenes; dialkyl ethers such as dicaprylylether; linear or cyclic silicone oils such as preferably cyclomethicone (octamethylcyclotetrasiloxane; cetyldimethicone, hexamethylcyclotrisiloxane, polydimethylsiloxane, poly(methylphenylsiloxane) and mixtures thereof.

Other fatty components which can advantageously be incorporated in topical compositions of the present invention are isoeikosane; neopentylglycoldiheptanoate; propylenglycol-dicaprytate/dicaprate; caprylic/capric/diglycerylsuccinate; butyleneglycol caprylat/caprat; C₁₂₋₁₃-alkyllactate; di-C₁₂₋₁₃ alkyltartrate; triisostearin; dipentaerythrityl hexacaprylat/hexacaprate; propylenglycolmonoisostearate; tricaprylin; dimethylisosorbid. Especially beneficial is the use of mixtures C₁₂₋₁₅-alkylbenzoate and 2-ethylhexylisostearate, mixtures C₁₂₋₁₅alkylbenzoate and isotridecylisononanoate as well as mixtures of C₁₂₋₁₅-alkylbenzoate, 2-ethylhexylisostearate and isotridecylisononanoate.

The oily phase of the compositions of the present invention can also contain natural vegetable or animal waxes such as bee wax, china wax, bumblebee wax and other waxes of insects as well as shea butter and cocoa butter.

A moisturizing agent may be incorporated into a topical composition of the present invention to maintain hydration or rehydrate the skin. Moisturizers that prevent water from evaporating from the skin by providing a protective coating are called emollients. Additionally an emollient provides a softening or soothing effect on the skin surface and is generally considered safe for topical use. Preferred emollients include mineral oils, lanolin, petrolatum, capric/caprylic triglyceraldehydes, cholesterol, silicones such as dimethicone, cyclomethicone, almond oil, jojoba oil, avocado oil, castor oil, sesame oil, sunflower oil, coconut oil and grape seed oil, cocoa butter, olive oil aloe extracts, fatty acids such as oleic and stearic, fatty alcohols such as cetyl and hexadecyl (ENJAY), diisopropyl adipate, hydroxybenzoate esters, benzoic acid esters of C₉₋₁₅-alcohols, isononyl iso-nonanoate, ethers such as polyoxypropylene butyl ethers and polyoxypropylene cetyl ethers, and C₁₂₋₁₅-alkyl benzoates, and mixtures thereof. The most preferred emollients are hydroxybenzoate esters, aloe vera, C₁₂₋₁₅-alkyl benzoates, and mixtures thereof. An emollient is present in an amount of about 1 wt.% to about 20 wt.% of the total weight of the composition. The preferred amount of emollient is about 2 wt.% to about 15 wt.%, and most preferably about 4 wt.% to about 10 wt.%.

Moisturizers that bind water, thereby retaining it on the skin surface are called humectants. Suitable humectants can be incorporated into a topical composition of the present invention such as glycerin, polypropylene glycol, 1,2-pentandiol, polyethylene glycol, lactic acid, pyrrolidone carboxylic acid, urea, phospholipids, collagen, elastin, ceramides, lecithin sorbitol, PEG-4, and mixtures thereof. Additional suitable moisturizers are polymeric moisturizers of the family of water soluble and/ or swellable/ and/ or with water gelating polysaccharides such as hyaluronic acid, chitosan and/or a fucose rich polysaccharide which is e.g. available as Fucogel®1000 (CAS-Nr. 178463-23-5) by SOLABIA S. One or more humectants are optionally present at about 0.5 wt.% to about 8 wt.% in a composition of the present invention, preferably about 1 wt.% to about 5 wt.%.

The aqueous phase of the topical compositions of the present invention can contain the usual cosmetic additives such as alcohols, especially lower alcohols, preferably ethanol and/ or isopropanol, low diols or polyols and their ethers, preferably propyleneglycol, glycerin, ethyleneglycol, ethyleneglycol monoethyl- or monobutylether, propyleneglycol monomethyl- or -monoethyl- or -monobutylether, diethyleneglycol monomethyl- or - monoethylether and analogue products, polymers, foam stabilizers; electrolytes and especially one or more thickeners. Thickeners that may be used in formulations of the present invention to assist in making the consistency of a product suitable include carbomer, siliciumdioxide, magnesium and/ or aluminum silicates, beeswax, stearic acid, stearyl alcohol polysaccharides and their derivatives such as xanthan gum, hydroxypropyl cellulose, polyacrylamides, acrylate crosspolymers preferably a carbomer, such as carbopole^{®} of type 980, 981, 1382, 2984, 5984 alone or mixtures thereof. Suitable neutralizing agents which may be included in the composition of the present invention to neutralize components such as e.g. an emulsifier or a foam builder/stabilizer include but are not limited to alkali hydroxides such as a sodium and potassium hydroxide; organic bases such as diethanolamine (DEA), triethanolamine (TEA), aminomethyl propanol, and mixtures thereof; amino acids such as arginine and lysine and any combination of any foregoing. The neutralizing agent can be present in an amount of about 0.01 wt.% to about 8 wt.% in the composition of the present invention, preferably, 1 wt.% to about 5 wt.%.

The addition of electrolytes into the composition of the present invention may be necessary to change the behavior of a hydrophobic emulsifier. Thus, the emulsions/microemulsions of this invention may contain preferably electrolytes of one or several salts including anions such as chloride, sulfates, carbonate, borate and aluminate, without being limited thereto. Other suitable electrolytes can be on the basis of organic anions such as, but not limited to, lactate, acetate, benzoate, propionate, tartrate and citrate. As cations preferably ammonium, alkylammonium, alkali- or alkaline earth metals, magnesium-, iron- or zinc-ions are selected. Especially preferred salts are potassium and sodium chloride, magnesium sulfate, zinc sulfate and mixtures thereof. Electrolytes can be present in an amount of about 0.01 wt.% to about 8 wt.% in the composition of the present invention.

The topical compositions of the invention can preferably be provided in the form of a lotion, a thickened lotion, a gel, a cream, a milk, an ointment, a powder or a solid tube stick and can be optionally be packaged as an aerosol and can be provided in the form of a mousse, foam or a spray. The compositions according to the invention can also be in the form of a suspension or dispersion in solvents or fatty substances, or alternatively in the form of an emulsion or microemulsion (in particular of O/W or W/O type, O/W/O or W/O/W-type), such as a cream or a milk, a vesicular dispersion, in the form of an ointment, a gel, a solid tube stick or an aerosol mousse. The emulsions can also contain anionic, nonionic, cationic or amphoteric surfactants.

According to the invention for preparing the compositions the active ingredients can be used as such or in an encapsulated form, for example in a liposomal form. Liposomes are preferably formed with lecithins with or without addition of sterols or phytosterols. The encapsulation of the active ingredients can be alone or together with other active ingredients.

In the topical compositions of the invention, the protein hydrolysates as defined above are contained in an amount of preferably 0.0001 wt.-% to about 50 wt.-%, based on the total weight of the composition. More preferably, the protein hydrolysates are contained in the compositions in an amount of about 0.01 wt.-% to about 20 wt.-%, more preferably in an amount of about 0.1 wt.-% to about 5 wt.%, in particular in an amount of about 0.1 to 3 wt.-%, and even more preferred in an amount of 0.3 to 1% based on the total amount of the composition. For particular fortified cosmetic products delivering stronger or maximized benefits it is preferred to use between 0.2 to 5 wt.-% and particular preferred to use 1 to 2 wt.-%. All percentage values are based on dry matter protein hydrolysate.

In case that the topical composition of the invention contains a further active ingredient, this further active ingredient is contained in an amount of preferably 0.0001 wt.% to about 50 wt.-%, based on the total weight of the composition. More preferably, the further active ingredient is contained in the composition in an amount of about 0.01 wt.% to about 20 wt.-%, more preferably in an amount of about 0.01 wt.-% to about 1 wt.-%, in particular in an amount of about 0.1 wt.%, based on the total amount of the composition.

Regarding the kind of the topical preparation and the preparation of the topical preparations as well as for further suitable additives, it can be referred to the pertinent literature, e.g. to Novak G.A., Die kosmetischen Präparate - Band 2, Die kosmetischen Präparate - Rezeptur, Rohstoffe, wissenschaftliche Grundlagen (Verlag für Chem. Industrie H. Ziolkowski KG, Augsburg).

The reduced or absent potential to cause adverse allergic reactions of the protein hydrolysate ingredient as well as for the cosmetic formulation for which it has been used can be measured using analytical kits. In general these kits are designed to detect certain peptide or protein fragments, which are known to cause adverse reactions in foodstuffs. They are commercially available, e.g. from Tepnel Biosystems Ltd. in Flintshire/UK.

### Examples

In the following examples protein hydrolysates are used which were obtained by a process as described in example 1 of WO 02/45523, using a suitable substrate and adapting the process conditions to the substrate as disclosed in WO 02/45523 and known to a skilled person, if necessary.

### Examples 1 -3 : Moisturizing Skin Lotions

| | **1** | **2** | **3** |
|---|---|---|---|
| **Ingredients** | **% (w/w)** | **% (w/w)** | **% (w/w)** |
| Water | Ad. 100 | Ad. 100 | Ad. 100 |
| D- Panthenol | 0.20 | 0.30 | 0.40 |
| **Casein Hydrolysate** | **0.20** | **0.50** | **0.80** |
| Propylene Glycol | 5.00 | 5.00 | 5.00 |
| Acrylates/C10-30 Alkyl Acrylate Crosspolymer | 0.50 | 0.50 | 0.50 |
| Sodium Hydroxide 30% | 0.40 | 0.40 | 0.40 |
| Disodium EDTA | 0.10 | 0.10 | 0.10 |
| Squalane | 2.00 | 2.00 | 2.00 |
| Phenoxyethanol & Methylparaben & Ethylparaben & Propylparaben & Butylparaben & Isopropylparaben | 0.80 | 0.80 | 0.80 |
| Coco-Caprylate/Caprate | 4.00 | 4.00 | 4.00 |
| BHT | 0.05 | 0.05 | 0.05 |
| Tocopheryl Acetate | 0.10 | 0.20 | 0.30 |
| Cyclomethicone | 3.00 | 3.00 | 3.00 |
| Glycerin | 3.00 | 3.00 | 3.00 |
| Sodium Hydroxide 10% | q.s. | q.s. | q.s. |
| Fragrance | q.s. | q.s. | q.s. |
| Polysorbate 20 | 0.80 | 0.80 | 0.80 |

### Examples 4 - 6: Skin Toners

| | **4** | **5** | **6** |
|---|---|---|---|
| **Ingredients** | **%(w/w)** | **%(w/w)** | **% (w/w)** |
| Water | Ad. 100 | Ad. 100 | Ad. 100 |
| Polyquaternium-10 | 0.10 | 0.10 | 0.10 |
| D- Panthenol | 0.40 | 0.60 | 0.60 |
| Sodium Ascorbyl Phosphate | 0.30 | 0.40 | 0.20 |
| Niacinamid | 0.40 | 0.40 | 0.20 |
| **Soy Protein Hydrolysate** | **0.10** | **0.50** | **1.00** |
| Propylene Glycol | 4.00 | 4.00 | 4.00 |
| Glycerin | 3.00 | 3.00 | 3.00 |
| PEG-12 Dimethicone | 0.20 | 0.20 | 0.20 |
| Disodium EDTA | 0.10 | 0.10 | 0.10 |
| Phenoxyethanol & Methylparaben & Ethylparaben & Propylparaben & Butylparaben & Isopropylparaben | 0.80 | 0.80 | 0.80 |
| Sodium Hydroxide 10% | q.s. | q.s. | q.s. |
| Polysorbate 20 | 2.00 | 2.00 | 2.00 |
| Fragrance | q.s. | q.s. | q.s. |

### Examples 7 - 9: Facial creams for sensitive skin protection

| | **7** | **8** | **9**(fortified formula) |
|---|---|---|---|
| **Ingredients** | **% (w/w)** | **% (w/w)** | **% (w/w)** |
| Glyceryl Myristate | 5.00 | 5.00 | 5.00 |
| Cetyl Alcohol | 2.00 | 2.00 | 2.00 |
| Cetyl Phosphate | 2.00 | 2.00 | 2.00 |
| Isopropyl Myristate | 10.00 | 10.00 | 10.00 |
| Tocopheryl Acetate | 0.30 | 0.30 | 0.30 |
| Almond Oil | 2.00 | 2.00 | 2.00 |
| BHT | 0.03 | 0.03 | 0.03 |
| Phenoxyethanol & Methylparaben & Ethylparaben | 0.60 | 0.60 | 0.60 |
| & Propylparaben & Butylparaben & | | | |
| Isopropylparaben | | | |
| Tromethamine | 0.90 | 0.90 | 0.90 |
| Water | Ad. 100 | Ad. 100 | Ad. 100. |
| D-Panthenol | 0.20 | 0.20 | 0.20 |
| Disodium EDTA | 0.10 | 0.10 | 0.10 |
| Propylene Glycol | 4.00 | 4.00 | 4.00 |
| Polyacrylamide & C13-14 Isoparaffin & Laureth-7 | 2.00 | 2.00 | 2.00 |
| **Barley Protein Hydrolysate** | **0.10** | **0.30** | **0.90** |
| Triethanolamine | q.s. | q.s. | q.s. |
| Fragrance | q.s. | q.s. | q.s. |
| Polysorbate 20 | 0.80 | 0.80 | 0.80 |

### Examples 10 - 12: Conditioning Shampoos

| | **10** | **11** | **12** (intensive treatment shampoo) |
|---|---|---|---|
| **Ingredients** | **% (w/w)** | **% (w/w)** | **% (w/w)** |
| Sodium Laureth Sulfate (~28%) | 50.00 | 50.00 | 50.00 |
| PEG-7 Glyceryl Cocoate | 3.00 | 3.00 | 3.00 |
| Cocamidopropyl Betaine (~ 30%) | 5.00 | 5.00 | 5.00 |
| Methylchloroisothiazolinone & Methylisothiazolinone | 0.10 | 0.10 | 0.10 |
| Water | Ad. 100 | Ad. 100 | Ad. 100 |
| Polyquaternium-10 | 0.25 | 0.15 | 0.05 |
| D-Panthenol | 0.80 | 0.50 | 0.20 |
| **Casein Hydrolysate** | **0.10** | **0.30** | **1.00** |
| Sodium Chloride | 2.00 | 2.00 | 2.00 |
| Disodium EDTA | 0.10 | 0.10 | 0.10 |
| Phytantriol | 0.10 | 0.10 | 0.10 |
| Tocopheryl Acetate | 0.05 | 0.05 | 0.05 |
| Fragrance | q.s. | q.s. | q.s. |
| Polysorbate 20 | 0.80 | 0.80 | 0.80 |

### Examples 13 - 15: Intensive Hair Conditioner

| | **13** | **14** | **15** (extra care formula for heavily damaged hair) |
|---|---|---|---|
| **Ingredients** | **% (w/w)** | **% (w/w)** | **% (w/w)** |
| Simmondsia Chinensis (Jojoba) Seed Oil | 3.00 | 3.00 | 3.00 |
| Prunius Armeniaca (Apricot) Kernel Oil | 3.00 | 3.00 | 3.00 |
| Phenyl Trimethicone | 2.00 | 2.00 | 2.00 |
| C12-15 Alkyl Benzoate | 2.00 | 2.00 | 2.00 |
| Glyceryl Stearate SE | 2.00 | 2.00 | 2.00 |
| Tocopheryl Acetate | 0.20 | 0.20 | 0.20 |
| Cetearyl Alcohol | 1.60 | 1.60 | 1.60 |
| Aqua | Ad. 100 | Ad. 100 | Ad. 100 |
| D-Panthenol | 0.50 | 0.50 | 0.50 |
| Behentrimonium Chloride | 0.10 | 0.10 | 0.10 |
| **Casein Hydrolysate** | **0.20** | **0.50** | **1.00** |
| Propylene Glycol & Diazolidinyl Urea & Methylparaben | 1.00 | 1.00 | 1.00 |
| Fragrance | q.s. | q.s. | q.s. |
| Phytantriol | 0.10 | 0.10 | 0.10 |
| Polysorbate 20 | 1.00 | 1.00 | 1.00 |

### Example 16 - 18: Hair Sera

| | **16** | **17** | **18** |
|---|---|---|---|
| **Ingredients** | **% (w/w)** | **%(w/w)** | **%(w/w)** |
| Water | Ad. 100 | Ad.100 | Ad.100. |
| **Gluten Hydrolysate** | **0.20** | **0.50** | **1.00** |
| Ethanol | 8.00 | 8.00 | 8.00 |
| Isopropanol | 4.00 | 4.00 | 4.00 |
| Propylene Glycol | 5.00 | 5.00 | 5.00 |
| Niacinamid | 0.15 | 0.15 | 0.15 |
| D-Panthenol | 1.00 | 0.50 | 0.20 |
| PEG-12 Dimethicone | 0.20 | 0.20 | 0.20 |
| PEG-40 Hydrogenated Castor Oil | 2.00 | 2.00 | 2.00 |
| Phytantriol | 0.05 | 0.15 | 0.10 |
| Fragrance | q.s. | q.s. | q.s. |
| Retinyl Palmitate | 0.05 | 0.05 | 0.05 |
| Tocopheryl Acetate | 0.10 | 0.10 | 0.10 |
| NaOH 10% | q.s. | q.s. | q.s. |

## Claims

1. Cosmetic composition comprising a protein hydrolysate and a cosmetically acceptable excipient, wherein the protein hydrolysate has a high content of peptides with a terminal proline, wherein the protein hydrolysate is a protein hydrolysate which comprises peptides, wherein the molar fraction of peptides (%) carrying a carboxy terminal proline is more than two times higher than the molar fraction (%) of proline in the protein substrate used to generate the hydrolysate, and wherein the composition is a topical composition.

2. Cosmetic composition according to claim 1, wherein the molar fraction of peptides (%) carrying a carboxy terminal proline is at least three times the molar fraction (%) of proline in the protein substrate used to generate the hydrolysate.

3. Cosmetic composition according to claim 1 or 2, wherein the average peptide length of the peptides in the protein hydrolysate is from 3 to 9 amino acids.

4. Cosmetic composition according to any of claims 1 to 3, wherein at least 10% of the protein substrate is hydrolyzed, preferably wherein 20 to 90% of the protein substrate is hydrolyzed.

5. Cosmetic composition according to any of claims 1 to 4, wherein the protein hydrolysate is
- a whey hydrolysate which comprises peptides wherein the molar fraction of peptides carrying a carboxy terminal proline is at least 8%, preferably at least 15%, more preferably from 30 to 70% and/or
- a casein hydrolysate which comprises peptides wherein the molar fraction of peptide carrying a carboxy terminal proline is at least 25%, preferably at least 30% and more preferably less than 70% and/or
- a soy hydrolysate which comprises peptides wherein the molar fraction of peptide carrying a carboxy terminal proline is at least 20%, preferably from 30 to 70% and/or
- a gluten hydrolysate which comprises peptides wherein the molar fraction of peptide carrying a carboxy terminal proline is at least 20%, preferably at least 30%, advantageously less than 70% and/or
- a barley hydrolysate which comprises peptides wherein the molar fraction of peptide carrying a carboxy terminal proline is at least 20%, preferably at least 30%, advantageously less than 70%.

6. Cosmetic composition according to claim 1, wherein the protein hydrolysate is rich in tripeptides, wherein the tripeptides are rich in proline at one end of the peptide, wherein at least 20 molar%, preferably at least 25 molar%, more preferably at least 30 molar% of the peptides having a molecular weight of 200 to 2000 Dalton in the protein hydrolysate is present as tripeptide, wherein at least 20%, preferably at least 30%, more preferably at least 40% of the proline present in the starting protein is present in the tripeptides.

7. Cosmetic composition according to claim 6, wherein at least 30% of the tripeptides or preferably at least 35% of the tripeptides have a carboxy terminal proline.

8. Cosmetic composition according to any of claims 6 or 7, wherein at least 70 molar% of the peptides, preferably at least 75 molar% of the peptides which are present in the protein hydrolysate contain 2 to 7 amino acid residues (dipeptide to heptapeptide).

9. Cosmetic composition according to any of claims 1 to 8, wherein the composition contains the protein hydrolysate in a concentration of 0.0001 to 50 wt.-% based on the weight of the composition.

10. Cosmetic composition according to claim 9, wherein the protein hydrolysate is present in a concentration of 0.01 to 20 wt.-% based on the weight of the composition.

11. Cosmetic composition according to claim 10, wherein the protein hydrolysate is present in a concentration of 0.1 to 5 wt.-% based on the weight of the composition.

12. Cosmetic composition according to claim 10, wherein the protein hydrolysate is present in a concentration of 0.3 to 1 wt.-% based on the weight of the composition.

13. Cosmetic composition according to any of claims 1 to 12, which is a skin cleansing composition, a hair care composition, a sunscreen composition, an anti-aging composition or a nail care composition.

14. Use of a protein hydrolysate as defined in any of claims 1 to 8 for providing a cosmetic effect, wherein the cosmetic effect is beautifying the skin, treatment or prophylaxis of wrinkles, moisturizing the skin, the inhibition of senescence of skin cells or non-therapeutical treatment of photodamage.

## Patentansprüche

1. Kosmetische Zusammensetzung, umfassend ein Proteinhydrolysat und einen kosmetisch akzeptablen Hilfsstoff, wobei das Proteinhydrolysat einen hohen Gehalt an Peptiden mit einem terminalen Prolin aufweist, wobei das Proteinhydrolysat ein Proteinhydrolysat ist, das Peptide umfasst, wobei die Molfraktion von Peptiden (%), die ein Carboxy-terminales Prolin tragen, mehr als zweimal höher ist als die Molfraktion (%) von Prolin in dem Proteinsubstrat, das zur Erzeugung des Hydrolysats verwendet wird, und wobei die Zusammensetzung eine topische Zusammensetzung ist.

2. Kosmetische Zusammensetzung nach Anspruch 1, wobei die Molfraktion von Peptiden (%), die ein Carboxy-terminales Prolin tragen, mindestens das Dreifache der Molfraktion (%) von Prolin in dem Proteinsubstrat, das zur Erzeugung des Hydrolysats verwendet wird, beträgt.

3. Kosmetische Zusammensetzung nach Anspruch 1 oder 2, wobei die durchschnittliche Peptidlänge der Peptide in dem Proteinhydrolysat 3 bis 9 Aminosäuren beträgt.

4. Kosmetische Zusammensetzung nach einem der Ansprüche 1 bis 3, wobei mindestens 10 % des Proteinsubstrats hydrolysiert sind, wobei vorzugsweise 20 bis 90 % des Proteinsubstrats hydrolysiert sind.

5. Kosmetische Zusammensetzung nach einem der Ansprüche 1 bis 4, wobei das Proteinhydrolysat
- ein Molkenhydrolysat, das Peptide umfasst, wobei die Molfraktion von Peptiden, die ein Carboxy-terminales Prolin tragen, mindestens 8 %, vorzugsweise mindestens 15%, stärker bevorzugt 30 bis 70 % beträgt, und/oder
- ein Caseinhydrolysat, das Peptide umfasst, wobei die Molfraktion von Peptiden, die ein Carboxy-terminales Prolin tragen, mindestens 25 %, vorzugsweise mindestens 30 % und stärker bevorzugt weniger als 70 % beträgt, und/oder
- ein Sojahydrolysat, das Peptide umfasst, wobei die Molfraktion von Peptiden, die ein Carboxy-terminales Prolin tragen, mindestens 20 %, vorzugsweise 30 bis 70 % beträgt, und/oder
- ein Glutenhydrolysat, das Peptide umfasst, wobei die Molfraktion von Peptiden, die ein Carboxy-terminales Prolin tragen, mindestens 20 %, vorzugsweise mindestens 30 %, vorteilhafterweise weniger als 70 % beträgt, und/oder
- ein Gerstenhydrolysat ist, das Peptide umfasst, wobei die Molfraktion von Peptiden, die ein Carboxy-terminales Prolin tragen, mindestens 20 %, vorzugsweise mindestens 30 %, vorteilhafterweise weniger als 70 % beträgt.

6. Kosmetische Zusammensetzung nach Anspruch 1, wobei das Proteinhydrolysat reich an Tripeptiden ist, wobei die Tripeptide an einem Ende des Peptids reich an Prolin sind, wobei mindestens 20 mol-%, vorzugsweise mindestens 25 mol-%, stärker bevorzugt mindestens 30 mol-% der Peptide mit einem Molekulargewicht von 200 bis 2000 Dalton in dem Proteinhydrolysat als Tripeptid vorliegen, wobei mindestens 20 %, vorzugsweise mindestens 30 %, stärker bevorzugt mindestens 40 % des Prolins, das in dem Ausgangsprotein vorliegt, in den Tripeptiden vorliegen.

7. Kosmetische Zusammensetzung nach Anspruch 6, wobei mindestens 30 % der Tripeptide oder vorzugsweise mindestens 35 % der Tripeptide ein Carboxy-terminales Prolin aufweisen.

8. Kosmetische Zusammensetzung nach einem der Ansprüche 6 oder 7, wobei mindestens 70 mol-% der Peptide, vorzugsweise mindestens 75 mol-% der Peptide, die in dem Proteinhydrolysat vorliegen, 2 bis 7 Aminosäurereste enthalten (Dipeptid bis Heptapeptid).

9. Kosmetische Zusammensetzung nach einem der Ansprüche 1 bis 8, wobei die Zusammensetzung das Proteinhydrolysat in einer Konzentration von 0,0001 bis 50 Gew.-%, basierend auf dem Gewicht der Zusammensetzung, enthält.

10. Kosmetische Zusammensetzung nach Anspruch 9, wobei das Proteinhydrolysat in einer Konzentration von 0,01 bis 20 Gew.-%, basierend auf dem Gewicht der Zusammensetzung, vorliegt.

11. Kosmetische Zusammensetzung nach Anspruch 10, wobei das Proteinhydrolysat in einer Konzentration von 0,1 bis 5 Gew.-%, basierend auf dem Gewicht der Zusammensetzung, vorliegt.

12. Kosmetische Zusammensetzung nach Anspruch 10, wobei das Proteinhydrolysat in einer Konzentration von 0,3 bis 1 Gew.-%, basierend auf dem Gewicht der Zusammensetzung, vorliegt.

13. Kosmetische Zusammensetzung nach einem der Ansprüche 1 bis 12, welche eine Hautreinigungszusammensetzung, eine Haarpflegezusammensetzung, eine Sonnenschutzzusammensetzung, eine Anti-Aging-Zusammensetzung oder eine Nagelpflegezusammensetzung ist.

14. Verwendung eines Proteinhydrolysats, wie in einem der Ansprüche 1 bis 8 definiert, zur Bereitstellung einer kosmetischen Wirkung, wobei die kosmetische Wirkung Verschönerung der Haut, Behandlung oder Prophylaxe von Falten, Feuchthalten der Haut, die Inhibierung der Alterung von Hautzellen oder die nicht-therapeutische Behandlung von Lichtschädigung ist.

## Revendications

1. Composition cosmétique comprenant un hydrolysat de protéines et un excipient acceptable au plan cosmétique, dans laquelle l'hydrolysat de protéines a une teneur élevée en peptides ayant une proline terminale, dans laquelle l'hydrolysat de protéines est un hydrolysat de protéines qui comprend des peptides, dans laquelle la fraction molaire des peptides (%) portant une proline carboxy-terminale est supérieure d'un facteur de plus de deux à la fraction molaire (%) de proline dans le substrat de protéines utilisé pour générer l'hydrolysat, et dans laquelle la composition est une composition topique.

2. Composition cosmétique selon la revendication 1, dans laquelle la fraction molaire de peptides (%) portant une proline carboxy-terminale représente au moins trois fois la fraction molaire (%) de proline dans le substrat de protéines utilisé pour générer l'hydrolysat.

3. Composition cosmétique selon la revendication 1 ou 2, dans laquelle la taille peptidique moyenne des peptides dans l'hydrolysat de protéines est de 3 à 9 acides aminés.

4. Composition cosmétique selon l'une quelconque des revendications 1 à 3, dans laquelle au moins 10 % du substrat de protéines sont hydrolysés, de préférence dans laquelle 20 à 90 % du substrat de protéines sont hydrolysés.

5. Composition cosmétique selon l'une quelconque des revendications 1 à 4, dans laquelle l'hydrolysat de protéines est l'un des suivantes :
- un hydrolysat de petit-lait qui comprend des peptides, dans lequel la fraction molaire des peptides portant une proline carboxy-terminale est d'au moins 8 %, de préférence d'au moins 15 %, mieux encore de 30 à 70 % et/ou
- un hydrolysat de caséine qui comprend des peptides, dans lequel la fraction molaire de peptides portant une proline carboxy-terminale est d'au moins 25 %, de préférence d'au moins 30 % et, mieux encore, inférieure à 70 % et/ou
- un hydrolysat de soja qui comprend des peptides, dans lequel la fraction molaire de peptides portant une proline carboxy-terminale est d'au moins 20 %, de préférence de 30 à 70 % et/ou
- un hydrolysat de gluten qui comprend des peptides, dans lequel la fraction molaire de peptides portant une proline carboxy-terminale est d'au moins 20 %, de préférence d'au moins 30 %, de manière avantageuse inférieure à 70 % et/ou
- un hydrolysat d'orge qui comprend des peptides, dans lequel la fraction molaire de peptides portant une proline carboxy-terminale est d'au moins 20 %, de préférence d'au moins 30 %, de manière avantageuse inférieure à 70 %.

6. Composition cosmétique selon la revendication 1, dans laquelle l'hydrolysat de protéines est riche en tripeptides, dans laquelle les tripeptides sont riches en proline à une extrémité du peptide, dans laquelle au moins 20 % en molaire, de préférence au moins 25 % en molaire, mieux encore au moins 30 % en molaire des peptides ayant un poids moléculaire de 200 à 2000 daltons dans l'hydrolysat de protéines sont présents sous la forme de tripeptides, dans laquelle au moins 20 %, de préférence au moins 30 %, mieux encore au moins 40 % de la proline présente dans la protéine de départ sont présents dans les tripeptides.

7. Composition cosmétique selon la revendication 6, dans laquelle au moins 30 % des tripeptides ou, de préférence, au moins 35 % des tripeptides ont une proline carboxy-terminale.

8. Composition cosmétique selon l'une quelconque des revendications 6 ou 7, dans laquelle au moins 70 % en molaire des peptides, de préférence au moins 75 % en molaire des peptides qui sont présents dans l'hydrolysat de protéines contiennent 2 à 7 résidus d'acide aminé (dipeptide à heptapeptide).

9. Composition cosmétique selon l'une quelconque des revendications 1 à 8, dans laquelle la composition contient l'hydrolysat de protéines en concentration de 0,0001 à 50 % en poids par rapport au poids de la composition.

10. Composition cosmétique selon la revendication 9, dans laquelle l'hydrolysat de protéines est présent en concentration de 0,01 à 20 % en poids par rapport au poids de la composition.

11. Composition cosmétique selon la revendication 10, dans laquelle l'hydrolysat de protéines est présent en concentration de 0,1 à 5 % en poids par rapport au poids de la composition.

12. Composition cosmétique selon la revendication 10, dans laquelle l'hydrolysat de protéines est présent en concentration de 0,3 à 1 % en poids par rapport au poids de la composition.

13. Composition cosmétique selon l'une quelconque des revendications 1 à 12, qui est une composition de nettoyage de la peau, une composition de soin pour les cheveux, une composition de protection solaire, une composition de soin contre le vieillissement ou une composition de soin pour les ongles.

14. Utilisation d'un hydrolysat de protéines selon l'une quelconque des revendications 1 à 8, pour fournir un effet cosmétique, dans laquelle l'effet cosmétique est un embellissement de la peau, un traitement ou une prophylaxie des rides, une hydratation de la peau, l'inhibition de la sénescence des cellules cutanées ou un traitement non thérapeutique de lésions d'origine photonique.
